# EUROPÄISCHE PATENTSCHRIFT

(11) **EP 1 863 553 B1**
(45) Veröffentlichungstag und Bekanntmachung des Hinweises auf die Patenterteilung: **07.10.2015**
(21) Anmeldenummer: 06723240.5
(22) Anmeldetag: 06.03.2006
(51) Int. Cl.: A61M 13/00, A61B 17/34

(54) **GASINSUFFLATIONSVORRICHTUNG**
GAS INSUFFLATION DEVICE
INSUFFLATEUR DE GAZ

(30) Priorität: 04.03.2005 DE 102005010062
(43) Veröffentlichungstag der Anmeldung: 12.12.2007
(73) Patentinhaber: Karl Storz GmbH & Co. KG, 78532 Tuttlingen (DE)
(72) Erfinder: SEMM, Horst, 82031 Grünwald (DE)
(74) Vertreter: Hoefer & Partner Patentanwälte mbB
(86) Internationale Anmeldenummer: PCT/EP2006/002040
(87) Internationale Veröffentlichungsnummer: WO 2006/092336

(56) Entgegenhaltungen:
- WO-A-01/08573
- WO-A-96/01132
- WO-A-2004/032770
- WO-A1-95/14436
- WO-A1-2004/012613
- DE-U1- 9 109 909
- US-A- 5 533 977
- US-B1- 6 656 160
- PATENT ABSTRACTS OF JAPAN Bd. 1997, Nr. 10, 31. Oktober 1997 (1997-10-31) -& JP 09 140721 A (OLYMPUS OPTICAL CO LTD), 3. Juni 1997 (1997-06-03)

## Beschreibung

Die Erfindung bezieht sich auf eine Gasinsufflationsvorrichtung gemäß dem Oberbegriff des Hauptanspruchs.

Im einzelnen bezieht sich die Erfindung auf eine Gasinsufflationsvorrichtung mit einem Trokar, welcher eine rohrförmige Trokarhülse umfasst, welche an einem Trokarkörper befestigt ist, sowie mit einem Trokardorn, welcher durch den Trokarkörper in die Trokarhülse einführbar ist.

Eine Gasinsufflationsvorrichtung der genannten Art ist beispielsweise aus der DE 30 00 218 C1 vorbekannt. Sie dient in der minimal-invasiven Chirurgie dazu, ein Pneumoperitonium aufzubauen und durch diese Möglichkeit intra-abdominale Untersuchungen oder Eingriffe vorzunehmen.

Bei der minimal-invasiven Chirurgie (MIC) wird ein Trokar durch einen relativ kleinen Schnitt in das Abdomen eingeführt. Durch den Trokar können dann ein Endoskop, weitere optische Instrumente oder chirurgische Instrumente eingeführt werden. Hierdurch ergibt sich die Möglichkeit, ein Pneumoperitonium aufzubauen. Dies erfordert die Zuführung eines Gasvolumens in das Abdomen, welches ausreichend groß ist, um ein ausreichendes Operationsfeld zu schaffen und um auftretende Leckagen, beispielsweise bei einem Instrumentenwechsel, unverzüglich auszugleichen. Auch kann es erforderlich sein, beispielsweise bei Laserverschweißungen im Abdomen entsprechend große Gasmengen zuzuführen.

Aus dem Stand der Technik ist es bekannt, zwischen der Trokarhülse und dem Trokardorn einen Ringspalt auszubilden, durch welchen die Gaszufuhr erfolgt. Dieser Ringspalt ist jedoch vielfach nicht ausreichend, um hohe Gasflüsse von bis zu 45 oder mehr 1/min zu realisieren. Abgesehen von der vielfach geringen Querschnittsfläche des Ringspalts bildet dieser einen Strömungswiderstand, der bei größeren Gasvolumina ungünstig ist.

Die JP 09 140721 A beschreibt eine Gasinsufflationsvorrichtung mit einem Trokar, welcher eine rohrförmige Trokarhülse umfasst, in welche ein Trokardorn geführt ist. Zur Ausbildung geeigneter Strömungskanäle ist die Trokarhülse rohrförmig ausgebildet, während der Trokardorn mit einer Längsnut versehen ist, welche den Strömungskanal bildet. Eine ähnliche Konstruktion zeigt auch die WO 01/08573 A. Um die Ausströmung aus der Trokarhülse zu verbessern, ist diese mit Ausnehmungen versehen.

Die US 6 656 160 B1 zeigt ebenfalls kreisrohrförmige Trokarhülsen, in welchen Instrumente mit unterschiedlichen Querschnitten geführt sind, um eine ausreichende Fluidströmung zu ermöglichen.

Aus der WO 96/01132 A ist es bekannt, jeweils kreisrunde Querschnitte für die Trokarhülse und den Trokardorn vorzusehen, diese jedoch im Durchmesser stark abzustufen, um einen ringförmigen Strömungsquerschnitt bereitzustellen.

Die DE 91 09 909 U1 und die WO 2004/032770 A zeigen jeweils ganz allgemein den Aufbau von Trokaren, ohne auf das Strömungskanalproblem einzugehen.

Der Erfindung liegt die Aufgabe zugrunde, eine Gasinsufflationsvorrichtung der eingangs genannten Art zu schaffen, welche bei einfachem Aufbau und einfacher, kostengünstiger Herstellbarkeit die zuverlässige Zuführung auch großer Gasvolumina ermöglicht.

Erfindungsgemäß wird die Aufgabe durch die Merkmalskombination des Hauptanspruchs gelöst. Die Unteransprüche zeigen weitere vorteilhafte Ausgestaltungen der Erfindung.

Erfindungsgemäß ist somit vorgesehen, dass der Innenquerschnitt der Trokarhülse zur Ausbildung zumindest eines zum Durchführen von Gas ausgebildeten Strömungskanals geometrisch unterschiedlich zu dem kreisförmigen Außenquerschnitt des Trokardorns und Zwar nicht-rund ausgebildet ist.

Erfindungsgemäß ist somit vorgesehen, einen zusätzlichen Strömungskanal zu schaffen, der zwischen der Außenwandung des Trokardorns und der Innenwandung der Trokarhülse dadurch ausgebildet wird, dass sich die Querschnittsformen des Innenquerschnitts der Trokarhülse und des Außenquerschnitts des Trokardorns unterscheiden.

Erfindungsgemäß ist es somit möglich, die Trokarhülse, welche als dünnwandiges Rohr aus einem medizinisch geeigneten Stahl hergestellt ist, mit einem beliebigen Querschnitt zu versehen, beispielsweise elliptisch, linsenförmig, quadratisch, rautenförmig, in Form eines Dreiecks oder polygonal. Dabei versteht sich, dass die Außenkontur jeweils entsprechend abgerundet oder mit einem Radius versehen ist, um Verletzungen oder Beeinträchtigungen des Patienten zu vermeiden. Da der Trokardorn einen runden, kreisförmigen Außenquerschnitt aufweist, ergibt sich somit zumindest ein zusätzlicher Strömungskanal, der durch die Relation der Querschnitte der Trokarhülse und des Trokardorns definiert ist. Bevorzugt können auch mehrere Strömungskanäle vorgesehen sein. Es ist somit möglich, den freien Strömungsquerschnitt des zumindest einen Strömungskanals so zu dimensionieren und zu bemessen, dass ein ausreichendes Gasvolumen durchführbar ist.

Erfindungsgemäß erweist es sich somit als besonders günstig, dass lediglich eine Anpassung der Trokarhülse erforderlich ist. Dies ist in einfachster Weise möglich, da derartige Trokarhülsen über eine Matrize gezogen werden. Somit ist eine besonders kostengünstige Herstellungsweise gegeben. Von dem kreisförmigen Querschnitt des Trokardorns, der in Form von Instrumenten oder Optiken ausgebildet ist, braucht somit nicht abgewichen werden.

Erfindungsgemäß ist es somit möglich, Gasströme bzw. Gasvolumina von beispielsweise 1 bis 45 1/min störungsfrei und ohne Gegendruck zu insufflieren.

Erfindunsgemäß ist auch vorgesehen, dass der Trokardorn an seinem distalen Endbereich mit einem Einführende versehen ist, welches in seinem Querschnitt passend zu dem nicht-runden Innenquerschnitt der Trokarhülse ausgebildet ist. Wenn sich somit das Einführende bzw. der distale Endbereich des Trokardorns im distalen Mündungsbereich der Trokarhülse befindet, kann diese problemlos in das Abdomen eingeführt werden, ohne dass sich die Gefahr von Verletzungen für den Patienten ergibt und ohne dass der Operationsverlauf hierdurch gestört oder beeinträchtigt würde. Der distale Endbereich des Trokardorns kann somit mit Ansätzen versehen sein, die hinsichtlich ihrer Geometrie und Größe exakt dem zumindest einen Strömungskanal entsprechen und diesen während des Einführvorganges abdecken oder abdichten, um ein unerwünschtes Einschieben von Gewebe o. ä. zu vermeiden. Nach dem vollständigen Einbringen des Trokars wird dann der Trokardorn weiter vorgeschoben, wodurch sein distaler Endbereich mit dem Einführende sich aus dem distalen Endbereich der Trokarhülse entfernt, so dass der zumindest eine Strömungskanal freigegeben wird.

Der distale Endbereich des Trokardorns ist vorzugsweise in Form' einer Trokarspitze (kegelig oder dreikant) ausgebildet, wobei die Verdickungen oder Ansätze, die in oben beschriebener Weise zum Verschließen des zumindest einen Strömungskanals dienen, glatt und ohne Absätze an die Trokarspitze anschließen.

Erfindungsgemäß ist es möglich, die Trokarhülse symmetrisch oder asymmetrisch auszubilden. Es ergeben sich die unterschiedlichsten Querschnittsvarianten. Besonders vorteilhafte Ausgestaltungsformen sind dann gegeben, wenn der kreisförmige Querschnitt des Trokardorns in der Trokarhülse entsprechend geführt oder zentriert ist. Hierdurch lässt sich ein Verklemmen mit Sicherheit vermeiden.

Erfindungsgemäß ist die Trokarhülse nicht-rund ausgebildet, so wie dies oben stehend erörtert wurde, nämlich beispielsweise dreieckig, quadratisch oder in sonstiger Weise. Hierdurch wird eine Verdrehsicherheit gewährleistet, die zudem ein Verrutschen oder Gleiten der Trokarhülse während einer Operation verhindert oder erschwert, so dass sich ein zusätzlicher vorteilhafter Effekt ergibt.

Die Trokarhülse kann erfindungsgemäß distal entweder gerade oder abgeschrägt ausgebildet sein, so dass übliche Grundformen von Trokarhülsen zur Anwendung kommen können.

Um eine optimierte Zuführung von Gas zu gewährleisten, kann es besonders günstig sein, wenn der Trokarkörper mit einem Gasanschlussrohr versehen ist, welches im Wesentlichen direkt in den zumindest einen Strömungskanal mündet. Dabei kann es auch günstig sein, eine Einströmkammer o. ä. auszubilden, um einen strömungstechnisch günstigen Einlauf des Gases in den oder die Strömungskanäle vorzusehen. Hierzu trägt beispielsweise auch eine axiale Neigung des Gasanschlussrohres bei.

Im Folgenden wird die Erfindung anhand von Ausführungsbeispielen in Verbindung mit der Zeichnung beschrieben. Dabei zeigt:
- Fig. 1: eine schematische Seitenansicht eines Trokars mit Trokarhülse und Trokardorn in dreieckiger Ausgestaltung,
- Fig. 2: eine stirnseitige Ansicht der Anordnung gemäß Fig. 1,
- Fig. 3: eine vergrößerte Teilansicht des Endbereichs eines zugehörigen Trokardorns,
- Fig. 4: eine Ansicht analog zu Fig. 1 in quadratischer Ausgestaltung,
- Fig. 5: eine Frontansicht der in Fig. 4 gezeigten Anordnung,
- Fig. 6: einen vorderen Endbereich eines zugehörigen Trokardorns mit distalem Endbereich,
- Fig. 7: eine Seitenansicht eines weiteren Ausführungsbeispiels, analog Fig. 1 und 4, in elliptischer Ausgestaltung,
- Fig. 8: eine stirnseitige Ansicht der Anordnung der Fig. 7,
- Fig. 9: eine Ansicht eines zugehörigen Trokardorns mit distalem Endbereich passend zu den Ausgestaltungen der Fig. 7 und 8,
- Fig. 10: eine schematische Seitenansicht eines Ausführungsbeispiels eines erfindungsgemäßen Trokardorns, und
- Fig. 11: eine Anordnung, analog Fig. 1, 4 oder 7, im Schnitt mit Zuordnung des Trokardorns.

Erfindungsgemäß ist eine Trokarhülse 1 vorgesehen, welche mit einem Trokarkörper 2 verbunden ist, beispielsweise mittels einer nicht näher gezeigten Verschraubung. Dies entspricht dem Stand der Technik. An dem Trokarkörper 2 ist ein Gasanschlussrohr 6 befestigt, welches zur Einleitung von Gas dient.

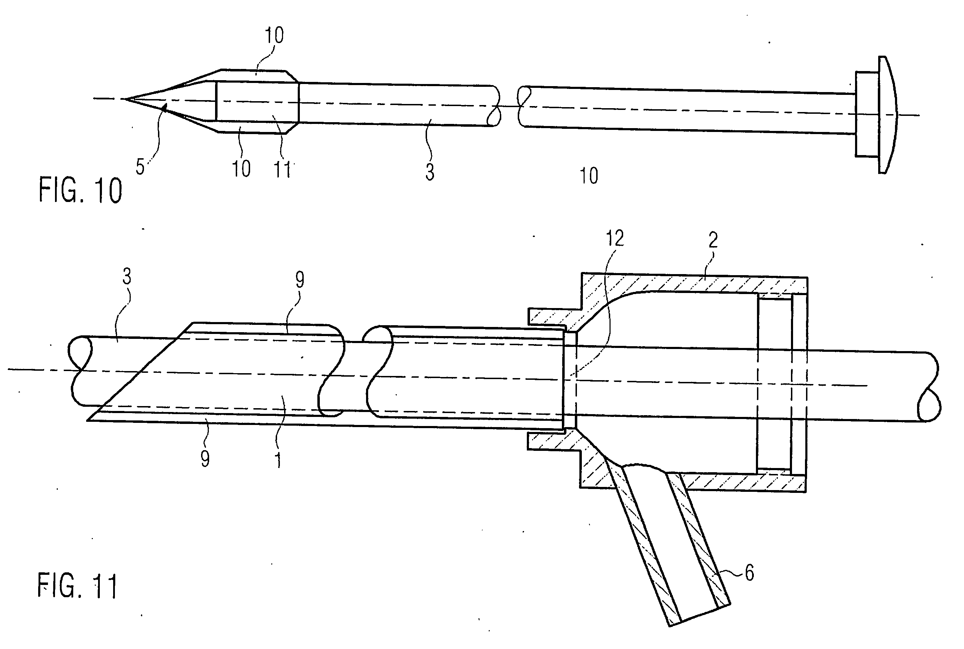

Wie die Fig. 11 zeigt, ist ein Trokardorn 3 (siehe Fig. 10) durch den Trokarkörper 2 in die Trokarhülse 1 einschiebbar. Auch dies entspricht dem Stand der Technik, auf die Darstellung der hierbei benötigten Membranen oder Dichtungsmittel wurde zum Zweck der Verdeutlichung verzichtet.

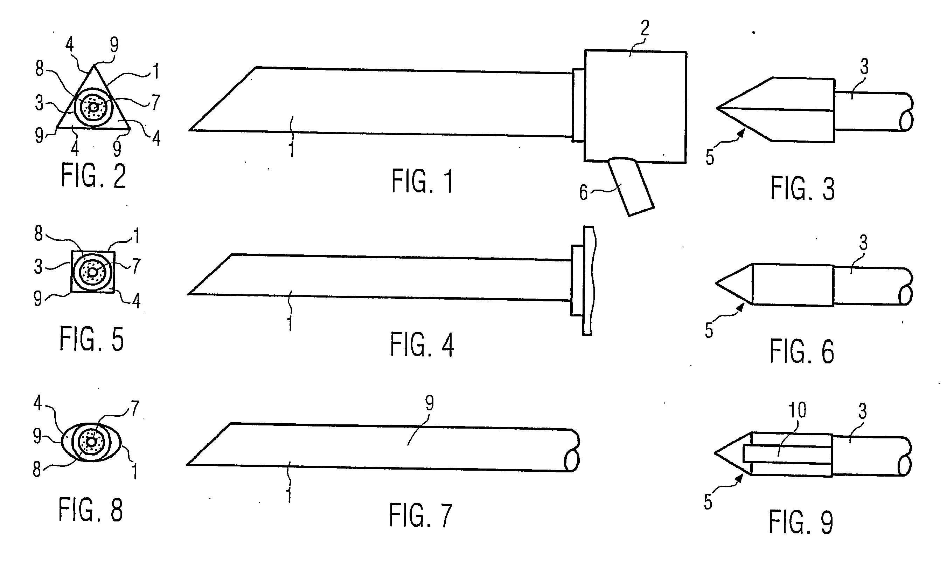

Der Trokardorn 3 weist einen runden, kreisförmigen Außenquerschnitt auf, so wie sich dies beispielsweise aus den Darstellungen der Fig. 3, 6, 9 und 10 ergibt. Der Trokardorn 3 kann, wie erwähnt, in unterschiedlichster Form ausgestaltet sein, so wie sich dies bei der minimal-invasiven Chirurgie ergibt. Die Fig. 2 zeigt beispielsweise eine Ausgestaltung mit einer Optik, welche eine Linse 7 und ein optisches Faserbündel 8 umfasst.

Die Fig. 1 bis 3 zeigen ein erstes Ausführungsbeispiel, bei welchem eine Trokarhülse 1 vorgesehen ist, welche einen dreieckigen Querschnitt aufweist (gleichseitiges Dreieck). Infolgedessen ergeben sich drei Ausbuchtungen 9, welche abgerundet sind und jeweils die Ecken des Dreieckes bilden. Durch die Ausbuchtungen 9 wird jeweils ein Strömungskanal 4 gebildet, so wie sich dies durch die Darstellung der Fig. 2 ergibt. In der dreieckigen Trokarhülse ist somit ein runder Trokardorn 3 geführt. Die Fig. 3 zeigt einen distalen Endbereich 5 eines Trokardorns 3, welcher ebenfalls einen dreieckigen Querschnitt aufweist und so in den dreieckigen Querschnitt der Trokarhülse 1 passt, dass beim Einschieben der Trokarhülse 1 deren Innenraum entsprechend abgedeckt oder abgedichtet ist, so dass sich kein Gewebe in den Strömungskanal 4 einschieben kann. Bei einem weiteren axialen Vorschub des Trokardorns 3 verlässt der distale Endbereich 5 das distale Ende der Trokarhülse 1, so dass die Strömungskanäle 4 freigegeben werden.

In den Fig. 4 bis 6 ist ein weiteres Ausführungsbeispiel gezeigt, bei welchem die Trokarhülse 1 einen quadratischen Querschnitt aufweist. Auch in diesem ist, wie sich aus Fig. 5 ergibt, der kreisrunde Querschnitt des Trokardorns 3 zentriert und geführt. Es versteht sich, dass der zugehörige Trokardorn 3 (siehe Fig. 6) mit einem distalen Endbereich 5 bzw. einer Spitze versehen ist, welche quadratisch ist und passend zu dem Innenraum des quadratischen Querschnitts der Trokarhülse 1 gestaltet ist.

Ein weiteres Ausführungsbeispiel zeigen die Fig. 7 bis 9. Hierbei ist der Querschnitt der Trokarhülse 1 linsenförmig ausgebildet, so dass sich zwei einander gegenüberliegende Ausbuchtungen 9 oder Ansätze ergeben, durch welche die beiden Strömungskanäle 4 gebildet werden. Auch hierbei führt der mittlere Bereich der linsenförmigen Trokarhülse 1 zentriert den Trokardorn 3. Aus der Darstellung der Fig. 9 ergibt sich, dass der distale Endbereich 5 entsprechend mit einem Ansatz 10 versehen ist, um beim Einschieben die beiden Strömungskanäle 4 des linsenförmigen Querschnitts entsprechend abzudichten oder zu verdecken. Die Fig. 10 zeigt in vergrößerter Darstellung die Ansicht der Fig. 9 (um 90° gedreht). Hierbei sind nochmals die beiden Ansätze 10 besonders deutlich dargestellt.

Die Fig. 11 zeigt in vergrößerter Darstellung im Schnitt das Ausführungsbeispiel der Fig. 7 bis 10. Hierbei ergibt sich insbesondere, dass die Gase durch das Gasanschlussrohr 6 in den Innenraum des Trokarkörpers 2 so einleitbar sind, dass diese in die Strömungskanäle 4 (in Fig. 11 nicht gezeigt), welche durch die Ausbuchtungen 9 gebildet werden, ungehindert einströmen können. Hierzu ist eine Einströmkammer 12 in schematischer Weise in Fig. 11 gezeigt.

### Bezugszeichenliste

- 1: Trokarhülse
- 2: Trokarkörper
- 3: Trokardorn
- 4: Strömungskanal
- 5: Distaler Endbereich
- 6: Gasanschlussrohr
- 7: Linse
- 8: Faserbündel
- 9: Ausbuchtung
- 10: Ansatz
- 11: Teilbereich
- 12: Einströmkammer

## Patentansprüche

1. Gasinsufflationsvorrichtung mit einem Trokar, welcher eine rohrförmige Trokarhülse (1) umfasst, welche an einem Trokarkörper (2) befestigt ist, sowie mit einem im Querschnitt kreisförmigen Trokardorn (3), welcher durch den Trokarkörper (2) in die Trokarhülse (1) einführbar ist, **dadurch gekennzeichnet, dass** der Innenquerschnitt der Trokarhülse (1) zur Ausbildung zumindest eines zum Durchführen von Gas ausgebildeten Strömungskanals (4) geometrisch unterschiedlich zu dem kreisförmigen Außenquerschnitt des Trokardorns (3) und Zwar nicht-rund ausgebildet ist, und dass der Trokardorn (3) an seinem distalen Endbereich (5) mit einem Einführende versehen ist, welches in seinem Querschnitt passend zu dem nicht-runden Innenquerschnitt der Trokarhülse (1) ausgebildet ist, um den zumindest einen Strömungskanal (4) während des Einführvorganges des Trokars abzudecken oder abzudichten.

2. Vorrichtung nach Anspruch 1, **dadurch gekennzeichnet, dass** der distale Endbereich (5) in Form einer Trokarspitze ausgebildet ist.

3. Vorrichtung nach einem der Ansprüche 1 bis 2, **dadurch gekennzeichnet, dass** die Trokarhülse (1) mit einem asymmetrischen Querschnitt versehen ist.

4. Vorrichtung nach einem der Ansprüche 1 bis 2, **dadurch gekennzeichnet, dass** die Trokarhülse (1) mit einem symmetrischen Querschnitt versehen ist.

5. Vorrichtung nach einem der Ansprüche 1 bis 3, **dadurch gekennzeichnet, dass** die Trokarhülse (1) im Querschnitt linsenförmig ausgebildet ist.

6. Vorrichtung nach einem der Ansprüche 1 bis 3, **dadurch gekennzeichnet, dass** die Trokarhülse (1) im Wesentlichen quadratisch ausgebildet ist.

7. Vorrichtung nach einem der Ansprüche 1 bis 3, **dadurch gekennzeichnet, dass** die Trokarhülse (1) im Wesentlichen rautenförmig ausgebildet ist.

8. Vorrichtung nach einem der Ansprüche 1 bis 3, **dadurch gekennzeichnet, dass** die Trokarhülse (1) im Wesentlichen in Form eines Dreiecks ausgebildet ist.

9. Vorrichtung nach einem der Ansprüche 1 bis 3, **dadurch gekennzeichnet, dass** die Trokarhülse (1) im Wesentlichen polygonal ausgebildet ist.

10. Vorrichtung nach einem der Ansprüche 1 bis 9, **dadurch gekennzeichnet, dass** die Trokarhülse (1) distal gerade ausgebildet ist.

11. Vorrichtung nach einem der Ansprüche 1 bis 9, **dadurch gekennzeichnet, dass** die Trokarhülse (1) distal abgeschrägt ausgebildet ist.

12. Vorrichtung nach einem der Ansprüche 2 bis 11, **dadurch gekennzeichnet, dass** der Trokarkörper (2) mit einem Gasanschlussrohr (6) versehen ist, welches im Wesentlichen direkt in den zumindest einen Strömungskanal (4) mündet.

13. Vorrichtung nach Anspruch 12, **dadurch gekennzeichnet, dass** das Gasanschlussrohr (6) in eine Einströmkammer (12) mündet, welche in den zumindest einen Strömungskanal (4) mündet.

## Claims

1. Gas insufflation device comprising a trocar, which includes a tubular trocar sleeve (1), which is fixed to a trocar body (2), and a trocar pin (3) having a circular cross section, which is insertable through the trocar body (2) into the trocar sleeve (1), **characterized in that** the inner cross section of the trocar sleeve (1) for forming a flow duct (4) formed for conducting of gas, is designed in a at least geometrically different manner from the circular outer cross section of the trocar pin (3), i.e. not round, and that the trocar pin (3) is provided with an insertion end at its distal end portion (5), which is designed in a cross section adapted to the non-round inner cross section of the trocar sleeve (1), in order to cover or to seal the at least one flow duct (4) during the introduction operation of the trocar.

2. Device of claim 1, **characterized in that** the distal end portion (5) forms a trocar tip.

3. Device of one of claims 1 to 2, **characterized in that** the trocar sleeve (1) is provided in an asymmetrical cross section.

4. Device of one of claims 1 to 2, **characterized in that** the trocar sleeve (1) is provided in a symmetrical cross section.

5. Device of one of claims 1 to 3, **characterized in that** the trocar sleeve (1) is formed in a lenticular shaped cross section.

6. Device of one of claims 1 to 3, **characterized in that** the trocar sleeve (1) is substantially square-shaped.

7. Device of one of claims 1 to 3, **characterized in that** the trocar sleeve (1) is substantially rhombic-shaped.

8. Device of one of claims 1 to 3, **characterized in that** the trocar sleeve (1) substantially forms a triangle.

9. Device of one of claims 1 to 3, **characterized in that** the trocar sleeve (1) is substantially polygonal-shaped.

10. Device of one of claims 1 to 9, **characterized in that** the trocar sleeve (1) is distally formed straight.

11. Device of one of claims 1 to 9, **characterized in that** the trocar sleeve (1) is distally formed chamfered.

12. Device of one of claims 1 to 11, **characterized in that** the trocar body (2) is provided with a gas connecting pipe (6), which substantially leads directly to at least one flow duct (4).

13. Device of claim 12, **characterized in that** the gas connecting pipe (6) leads to an inlet chamber (12), which leads at least to one flow duct (4).

## Revendications

1. Dispositif insufflateur de gaz comprenant un trocart, qui comprend un manchon de trocart (1) de forme tubulaire, qui est fixé au niveau d'un corps de trocart (2), comprenant également un mandrin de trocart (3) de forme circulaire dans la section transversale, lequel peut être introduit dans le manchon de trocart (1) par le corps de trocart (2), **caractérisé en ce que** la section transversale intérieure du manchon de trocart (1) est réalisée de manière à présenter une forme géométrique différente par rapport à la section transversale extérieure de forme circulaire du mandrin de trocart (3), à savoir une forme qui n'est pas ronde, afin de réaliser au moins un canal d'écoulement (4) réalisé afin d'acheminer du gaz, et **en ce que** le mandrin de trocart (3) est pourvu, au niveau de sa zone d'extrémité (5) distale, d'une extrémité d'introduction, qui est réalisée de manière à s'adapter dans sa section transversale à la section transversale intérieure du manchon de trocart (1) qui n'est pas ronde afin de recouvrir ou de rendre étanche, le au moins un canal d'écoulement (4) au cours de l'opération d'introduction du trocart.

2. Dispositif selon la revendication 1, **caractérisé en ce que** la zone d'extrémité (5) distale est réalisée sous la forme d'une pointe de trocart.

3. Dispositif selon l'une quelconque des revendications 1 à 2, **caractérisé en ce que** le manchon de trocart (1) est pourvu d'une section transversale asymétrique.

4. Dispositif selon l'une quelconque des revendications 1 à 2, **caractérisé en ce que** le manchon de trocart (1) est pourvu d'une section transversale symétrique.

5. Dispositif selon l'une quelconque des revendications 1 à 3, **caractérisé en ce que** le manchon de trocart (1) est réalisé de manière à présenter dans la section transversale une forme de lentille.

6. Dispositif selon l'une quelconque des revendications 1 à 3, **caractérisé en ce que** le manchon de trocart (1) est réalisé sensiblement de manière carrée.

7. Dispositif selon l'une quelconque des revendications 1 à 3, **caractérisé en ce que** le manchon de trocart (1) est réalisé sensiblement de manière à présenter une forme de losange.

8. Dispositif selon l'une quelconque des revendications 1 à 3, **caractérisé en ce que** le manchon de trocart (1) est réalisé sensiblement sous la forme d'un triangle.

9. Dispositif selon l'une quelconque des revendications 1 à 3, **caractérisé en ce que** le manchon de trocart (1) est réalisé sensiblement de manière polygonale.

10. Dispositif selon l'une quelconque des revendications 1 à 9, **caractérisé en ce que** le manchon de trocart (1) est réalisé de manière rectiligne distalement.

11. Dispositif selon l'une quelconque des revendications 1 à 9, **caractérisé en ce que** le manchon de trocart (1) est réalisé de manière biseautée distalement.

12. Dispositif selon l'une quelconque des revendications 2 à 11, **caractérisé en ce que** le corps de trocart (2) est pourvu d'un tuyau de raccordement de gaz (6), qui débouche sensiblement directement dans le au moins un canal d'écoulement (4).

13. Dispositif selon la revendication 12, **caractérisé en ce que** le tube de raccordement de gaz (6) débouche dans une chambre de flux entrant (12), laquelle débouche dans le au moins un canal d'écoulement (4).
